(19) **Europäisches Patentamt**
**European Patent Office**
**Office européen des brevets**

(11) **EP 4 272 788 B1**

(12) **EUROPEAN PATENT SPECIFICATION**

(45) Date of publication and mention
of the grant of the patent:
**04.02.2026 Bulletin 2026/06**

(21) Application number: **20967473.8**

(22) Date of filing: **29.12.2020**

(51) International Patent Classification (IPC):
***A61M 16/00*** *(2006.01)*

(52) Cooperative Patent Classification (CPC):
(C-Sets available)
**A61M 16/024;** A61M 16/204; A61M 16/205;
A61M 2016/0027; A61M 2016/0036;
A61M 2205/3334; A61M 2205/505; A61M 2230/42;
A61M 2230/46                                    (Cont.)

(86) International application number:
**PCT/CN2020/141088**

(87) International publication number:
**WO 2022/141125 (07.07.2022 Gazette 2022/27)**

(54) **RESPIRATORY SUPPORT DEVICE**

ATEMUNTERSTÜTZUNGSVORRICHTUNG

DISPOSITIF D'ASSISTANCE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.11.2023 Bulletin 2023/45**

(73) Proprietors:
• **Zhongda Hospital Southeast University
Nanjing, Jiangsu 210009 (CN)**
• **Shenzhen Mindray Bio-Medical Electronics Co.,
Ltd.
Shenzhen, Guangdong 518057 (CN)**

(72) Inventors:
• **LIU, Ling
Shenzhen, Guangdong 518057 (CN)**
• **LIU, Jinglei
Shenzhen, Guangdong 518057 (CN)**
• **YANG, Yi
Shenzhen, Guangdong 518057 (CN)**

• **ZHOU, Xiaoyong
Shenzhen, Guangdong 518057 (CN)**
• **QIU, Haibo
Shenzhen, Guangdong 518057 (CN)**
• **HUANG, Zhiwen
Shenzhen, Guangdong 518057 (CN)**
• **XIE, Jianfeng
Shenzhen, Guangdong 518057 (CN)**
• **ZHU, Feng
Shenzhen, Guangdong 518057 (CN)**
• **SUN, Qin
Shenzhen, Guangdong 518057 (CN)**

(74) Representative: **KIPA AB
Drottninggatan 11
252 21 Helsingborg (SE)**

(56) References cited:
**EP-A1- 3 238 110           WO-A1-2017/077417
CN-A- 102 451 506       CN-A- 107 209 797
CN-A- 107 519 562       CN-A- 109 718 442
US-A1- 2009 229 611   US-A1- 2012 000 470
US-A1- 2013 255 685   US-A1- 2014 318 541**

(52) Cooperative Patent Classification (CPC): (Cont.)

C-Sets
A61M 2230/42, A61M 2230/005;
A61M 2230/46, A61M 2230/005

**Description**

TECHNICAL FIELD

The disclosure relates to the technical field of medical devices, and more particularly to a respiratory support device and a control method therefor, and a storage medium.

BACKGROUND

Human respiration refers to the periodic and rhythmic inhalation and exhalation of gas, absorption of oxygen and emission of carbon dioxide for gas exchange. When some patients are unable to breathe autonomously, mechanical ventilation can be used to help them to complete their respiration. For example, in cases where patients do not breathe autonomously, external devices, such as ventilators, can usually be used to provide respiratory support to patients.
At present, when using the respiratory support device, such as a ventilator or anesthesia machine, to ventilate patient, it is necessary to adjust ventilation parameters of the respiratory support device to values suitable for the patient. Currently, medical staff mainly rely on experience or esophageal pressure assistance to manually adjust the ventilation parameters. Due to subjective factors and limited energy of medical staff, it is difficult for them to achieve real-time monitoring. Therefore, manual adjustment can lead to a decrease in the accuracy and timeliness of ventilation parameter adjustment, putting the patient at risk of lung injury. A patent application EP3238110A1 provides teachings related to the technical field of the application.

SUMMARY

This disclosure provides a respiratory support device and a control method therefor, and a storage medium, aiming to reduce the risk of lung injury and solve technical problem, such as inconvenience and untimely manual adjustment of ventilation parameters of respiratory support device. The invention is defined by the appended claims.
According to a first aspect, an embodiment not being part of this invention provides a control method for a respiratory support device, including:

obtaining a ventilation parameter configuration of the respiratory support device during ventilation for a patient, wherein the ventilation parameter configuration includes a set value for at least one ventilation parameter;
determining mechanical power according to the ventilation parameter configuration, wherein the mechanical power is configured to indicate power of mechanical ventilation, which acts on lung(s) or a respiratory system;
adjusting the ventilation parameter configuration according to the mechanical power, so as to enable the mechanical power determined after the adjustment to satisfy a preset condition.

According to a second aspect, an embodiment of this disclosure provides a respiratory support device, including:

a gas flow supply apparatus, which is configured to generate a ventilation gas flow;
a respiratory line, which is connected with the gas flow supply apparatus and configured to transmit the ventilation gas flow, which is generated by the gas flow supply apparatus, to an airway of a patient;
one or more processors, which work individually or collectively, so as to perform following steps:

obtaining a ventilation parameter configuration of the respiratory support device during ventilation for the patient, wherein the ventilation parameter configuration includes a set value for at least one ventilation parameter;
determining mechanical power according to the ventilation parameter configuration, wherein the mechanical power is configured to indicate power of mechanical ventilation, which acts on lung(s) or a respiratory system;
adjusting the ventilation parameter configuration according to the mechanical power, so as to enable the mechanical power determined after the adjustment to satisfy a preset condition.

According to a third aspect, an embodiment not being part of this invention provides a computer readable storage medium, which stores a computer program, and when the computer program is executed by a processor, the control method described above is implemented.
The embodiments of this disclosure provides a respiratory support device, which determine mechanical power according to the ventilation parameter configuration of the respiratory support device during ventilation of the patient, wherein the mechanical power is configured to indicate power of mechanical ventilation, which acts on lung(s) or a respiratory system; and adjust the ventilation parameter configuration according to the mechanical power, so as to enable the mechanical power determined after the adjustment to satisfy a preset condition. In this way, automatic adjustment of ventilation parameter configuration without the need for manual adjustment, is realized, the accuracy and timeliness of ventilation parameter configuration adjustment are improved, the risk of lung injury for patient is reduced or avoided, and the convenience and reliability of ventilation control for respiratory support device, are also improved.

It should be understood that the above general description and the following detailed description are only exemplary and explanatory, and do not limit the disclosure.

BRIEF DESCRIPTION OF THE DRAWINGS

In order to more clearly explain the embodiments of this disclosure, the following briefly introduces the drawings which are needed to be used in the description of the embodiments. It is obvious that the drawings in the following description are only some embodiments of this disclosure. For those skilled in the art, other drawings can be obtained from these accompanying drawings without paying any creative works.

FIG. 1 is a flowchart of a control method for a respiratory support device provided in an embodiment not being part of this invention.

FIG. 2 is a structural diagram for a respiratory support device provided in an embodiment of this disclosure;

FIG. 3 is a structural diagram for a ventilator provided in an embodiment of this disclosure.

DESCRIPTION OF THE PREFERRED EMBODIMENTS

The technical solutions in the embodiments of this disclosure are described clearly and completely below in combination with the drawings in the embodiments of this disclosure. Obviously, the described embodiments are just some of the embodiments of this disclosure, not all of them.

The flowchart shown in the attached diagram is only an example and does not necessarily include all content and operations/steps, nor does it have to be executed in the order described. For example, some operations/steps can also be decomposed, combined, or partially merged, so the actual order of execution may change according to the actual situation.

Some embodiments of this disclosure are described in detail below in combination with the accompanying drawings. The following embodiments and features in the embodiments may be combined with each other without conflict.

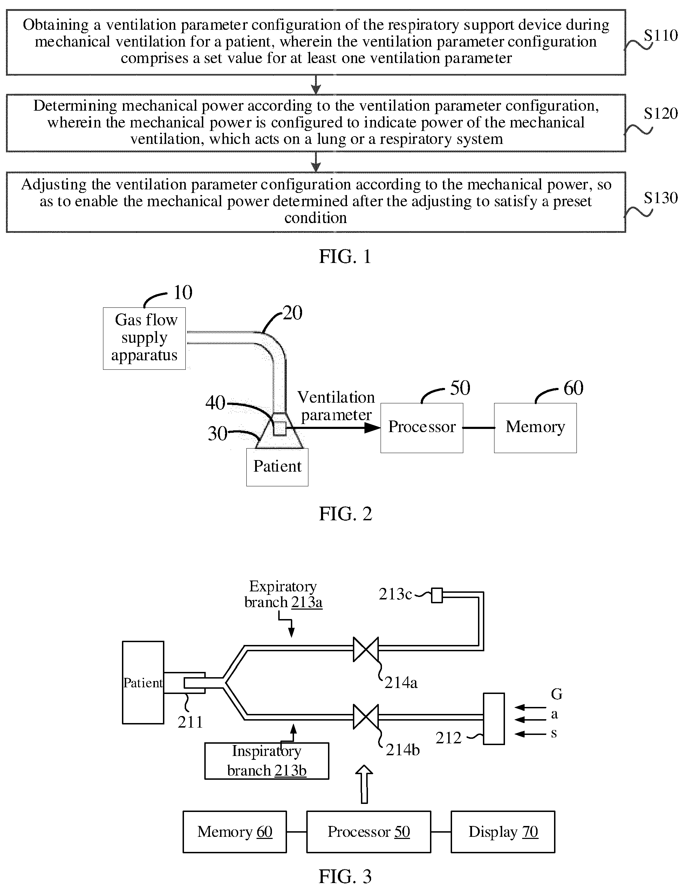

Please refer to FIG. 1, which is a flowchart of a control method for a respiratory support device provided in an embodiment not being part of this invention. The control method can be applied to a respiratory support device or a control device of the respiratory support device, for adjusting the ventilation parameter configuration and other processes of respiratory support device.

FIG. 2 is a scenario diagram for implementing the control method for the respiratory support device provided in an embodiment of this disclosure. As shown in FIG. 2, the respiratory support device includes a gas flow supply apparatus 10 and a respiratory line 20, which is connected with the gas flow supply apparatus 10 to transmit ventilation gas flow generated by the gas flow supply apparatus 10 to an airway of a patient.

For example, the respiratory support device further includes a patient interface 30, which includes a mask, a nasal mask, a nasal cannula, a tracheal intubation, and the likes, which are attached to the patient. Wherein, the gas flow supply apparatus 10 is connected to the patient interface 30 through the respiratory line 20, for transmitting the ventilation gas flow to the airway of the patient.

For example, the respiratory support device further includes a ventilation detection apparatus 40, which is arranged at the respiratory line or patient interface to detect ventilation parameters, which include a flow rate of the ventilation gas flow, an airway pressure, a respiratory frequency, a tidal volume, inspiratory time, respiratory system compliance or lung compliance, etc. It should be noted that the ventilation parameters can be either directly detected or calculated after detecting certain basic parameters.

For example, some ventilation parameters, such as ventilation volume, can also be obtained through devices other than respiratory support devices, such as monitors.

Specifically, the respiratory support device includes one or more processors 50, which work individually or collectively, so as to execute steps of the control method for the respiratory support device.

For example, the respiratory support device further includes a memory 60, and the processor 50 and memory 60 can be connected through a bus, such as I2C (Inter integrated Circuit) bus.

Wherein, the processor 50 is configured to execute a computer program stored in the memory 60, and to implement any control method of respiratory support device provided in an embodiment of this disclosure when executing the computer program.

The respiratory support device further includes a human-machine interaction apparatus, which includes a display for displaying positive end expiratory pressure when the respiratory support device ventilates the patient, as well as displaying patient state information, ventilation parameters, etc. The specific display content includes text, chart, number, color, waveform, character, etc., for intuitively displaying various types of information. In practical applications, the human-machine interaction apparatus further includes an input device, through which medical staff can set various parameters, as well as select and control a display interface of the display, to achieve information exchange between human and machine. This display can also be a touch display.

In some embodiments, the respiratory support device may be a ventilator or anesthesia machine, as explained in detail below.

In some embodiments, the respiratory support device can be a ventilator, which is an artificial mechanical ventilation device configured to assist or control the respiratory movement of the patient, so as to achieve gas exchange in the lung, reduce the work done by the patient in respiration, and facilitate the recovery of respiratory function. Please refer to FIG. 3. In some embodiments, the respiratory support device further includes a respiratory interface 211 (i.e., patient interface), a gas source interface 212, a respiratory loop (i.e., respiratory line), a respiratory assistance apparatus (i.e., gas flow supply apparatus), a ventilation detection apparatus for detecting ventilation parameters, a processor 50, a memory 60, and a display 70. The processor 50 determines a target positive end expiratory pressure according to ventilation parameters detected by the ventilation detection apparatus, so as to control the respiratory assistant apparatus to ventilate the patient according to the target positive end expiratory pressure.

The respiratory loop selectively connects the gas source interface 212 to the respiratory system of the patient. In some embodiments, the respiratory loop includes an expiratory branch 213a and an inspiratory branch 213b, which are connected between the respiratory interface 211 and a vent 213c to guide exhaled gas of the patient to the vent 213c. The vent 213c can be connected to external environment or a dedicated gas recovery apparatus. The gas source interface 212 is configured to connect to a gas source (not shown in the figure), which is configured to provide gas, which can usually be oxygen, air, etc. In some embodiments, the gas source can use a compressed gas cylinder or a central gas source to supply gas to the ventilator through the gas source interface 212, wherein the gas includes oxygen and air, etc. The gas source interface 212 includes conventional components such as a pressure gauge, pressure regulator, flow meter, pressure reducing valve, and air oxygen ratio control protection device, which are configured to control flow amount of various gases (such as oxygen and air). The inspiratory branch 213b is connected between the respiratory interface 211 and the gas source interface 212 to provide oxygen or air to the patient. For example, the gas from the gas source interface 212 is inputted into the inspiratory branch 213b and then into lungs of the patient through the respiratory interface 211. The respiratory interface 211 is configured to connect the patient to the respiratory loop. In addition to introducing the gas transmitted from the inspiratory branch 213b to the patient, the gas exhaled by the patient can also be introduced into the vent 213c through the expiratory branch 213a. According to the situation, the respiratory interface 211 can be a nasal cannula or a mask worn on the mouth and nose. The respiratory assistant apparatus is connected to the gas source interface 212 and the respiratory loop to control the delivery of gas provided by the external gas source to the patient through the respiratory loop. In some embodiments, the respiratory assistant apparatus may include an expiratory controller 214a and an inspiratory controller 214b. The expiratory controller 214a is arranged at the expiratory branch 213a, and configured to connect or close the expiratory branch 213a according to a control instruction, or to control the flow rate or pressure of exhaled gas of the patient. When specifically implemented, the expiratory controller 214a includes one or more devices that can control flow or pressure, such as an expiratory valve, one-way valve, flow controller, PEEP valve, etc. The inspiratory controller 214b is arranged at the inspiratory branch 213b, and configured to connect or close the inspiratory branch 213b according to a control instruction, or to control the flow rate or pressure of the outputted gas. When specifically implemented, the inspiratory controller 214b may include one or more devices that can control flow or pressure, such as an inspiratory valve, one-way valve, flow controller, PEEP valve, etc.

The memory 60 is configured to store data or programs, such as data acquired by a sensor or ventilation detection apparatus, as well as data generated by the processor 50 through calculation or image frames generated by the processor 50. The image frames can be 2D or 3D images, or the memory 60 can store a graphical user interface, one or more default image display settings, and programming instructions for the processor. The memory 60 can be a tangible and non-transient computer-readable medium, such as flash memory, RAM, ROM, EEPROM, etc.

In some embodiments, the processor 50 can also be configured to execute instructions or programs, control various control valves in the respiratory assistant apparatus, gas source interface 212, and/or respiratory loop, or process the received data to generate the required calculation or determination results, or generate visual data or graphics, and output the visual data or graphics to the display 70.

The above are some descriptions of the respiratory support device as a ventilator. It should be noted that FIG. 3 above is only an example of a ventilator, and it is not intended to limit the structure of a ventilator.

The control method can also be applied to the control device of respiratory support device, which can be in communicative connection with the respiratory support device to adjust the ventilation parameter configuration and other processes of the respiratory support device. For example, the control device can be at least one of a mobile phone, a tablet, a laptop, a desktop, a personal digital assistant, a wearable device, a remote controller, etc.

Please refer to FIG. 1. The control method for the respiratory support device in the embodiment not being part of this invention includes steps S110 to S130, which can be as follows.

In step S110, a ventilation parameter configuration of the respiratory support device during ventilation for the patient, is obtained; wherein the ventilation parameter configuration includes a set value for at least one ventilation parameter.

In the disclosure, the ventilation parameter includes at least one of the followings: positive end expiratory pressure (PEEP), respiratory rate (RR), tidal volume (VT), gas flow rate (Flow), inhaled oxygen concentration (FiO2), airway pressure (Paw), pressure rise time (Ramp), and inspiratory time (Ti).

Positive end expiratory pressure (PEEP) can increase the end expiratory lung volume (EELV), make the alveoli not easy to collapse at the end of expiration, increase the end expiratory Lung volume, increase difference between oxygen partial pressures of alveolar and arterial blood, promote the regression of interstitial and alveolar edema, thus improving the alveolar diffusion function and ventilation/blood flow ratio, and reducing intrapulmonary shunting to improve oxygenation and lung compliance.

Respiratory frequency, also expressed as f, can be a number of times a patient breathes in one minute. Tidal volume refers to a volume of gas inhaled or exhaled each time when breathing calmly. Tidal volume is related to age, gender, volume surface, respiration habit, and body metabolism. Tidal volume set in the disclosure can refer to the volume of gas inhaled, for example, the volume of gas inhaled by a patient at one time.

For example, the set value for the ventilation parameter during ventilation for a patient can be determined by at least one of the following methods. The set value for the ventilation parameter during ventilation for a patient can be determined according to a user setting operation, a pre-stored set value, and a preset determination logic.

For example, the respiratory support device provides a visual interface that displays set values of the ventilation parameter and allows the user to determine the set values of ventilation parameter through setting operation.

For example, the respiratory support device stores multiple set values for the ventilation parameter in advance, determines default set value(s), or displays corresponding visual marks of the multiple set values for the ventilation parameter on the visual interface for the user to select and confirm.

For example, determining the set value for ventilation parameter for patient according to a preset determination logic includes determining the set value for ventilation parameter for the patient according to the control method of the disclosure. Appropriate ventilation parameter configuration is very important for patient treatment. The control method of this disclosure can automatically adjust the set value for the ventilation parameter, such as positive end expiratory pressure dynamically, so that the adjusted set value can be suitable for the patient. The adjustment of the set value for the ventilation parameter can be adjustment of the set value for the ventilation parameter to adapt the patient, when the patient starts using the respiratory support device for ventilation, or can be dynamically adjustment of the set value for the ventilation parameter during the operation of the respiratory support device.

In some embodiments, the respiratory support device provides a visual interface that can be operated by the user to determine whether to turn on or off the automatic adjustment function of ventilation parameter configuration. When the automatic adjustment function of ventilation parameter configuration is activated, the control method of this disclosure automatically adjusts the set value for the ventilation parameter, such as positive end expiratory pressure dynamically.

In step 120, mechanical power is determined according to the ventilation parameter configuration, wherein the mechanical power is configured to indicate a power of mechanical ventilation which acts on the lungs or respiratory system.

Mechanical power represents the amount of power which is applied by respiratory support device to the patient. Under the same ventilation volume, it is hoped that the mechanical power can remain stable or at a smaller level, which can reduce lung injury.

During the mechanical ventilation, the settings of ventilation parameters, such as tidal volume (Vt), positive end expiratory pressure (PEEP), respiratory rate (RR), and respiratory mechanics of the patient (such as airway resistance and respiratory system compliance) under different mechanical ventilation modes all affect the mechanical power of mechanical ventilation on the lungs or respiratory system.

The control method of this disclosure obtains the ventilation parameter configuration of the respiratory support device during ventilation for a patient, determines the mechanical power according to the ventilation parameter configuration, and achieves real-time monitoring of mechanical power, so as to automatically adjust the mechanical ventilation parameter according to the monitoring result of mechanical power, thus ensuring the comfort and safety of clinical patient without the need for clinical adjustment of the doctor, and so as to automatically adjust the settings of mechanical ventilation parameter according to the guidance of mechanical power, thus reducing or avoiding the risk of ventilator-related lung injury for patient.

In some embodiments, the respiratory support device can determine mechanical power by providing ventilation parameters, such as pressure and flow rate, during ventilating the patient, wherein the pressure is real-time airway pressure of the patient and the flow rate is the gas flow rate.

For example, the airway pressure and the gas flow rate can be integrated within a preset unit time, or within one respiration cycle, or within a preset time cycle, to obtain the corresponding mechanical power $Power_{rs}$ with a unit of joules per minute (J/min).

For example, the complete formula for calculating the mechanical power can be as follows:

$$Power_{rs} = 0.098 \times RR \times \left\{ VT^2 \times \left[ \frac{1}{2C_{rs}} + RR \times \frac{(1+I:E)}{60 \times I:E} \times R_{aw} \right] + VT \times PEEP + \frac{1}{2} PEEP_{volume} \times PEEP \right\}.$$

The pressure and flow rate integration method can also be used for calculation, and its calculation formula can be as follows:

$$Power_{rs} = 0.098 \times RR \times \left( \int Paw \times Flow\ dt + \frac{1}{2} PEEP_{volume} \times PEEP \right).$$

Furthermore, the mechanical power can also be calculated by a simplified integral method formula as follows:

$$Power_{rs} = \int Paw \times Flow\ dt$$

wherein 0.098 is a constant value (0.098: $1cmH2O \times 1L/min = 0.098\ J/min$), and can also be set to other constant values according to actual requirements, RR represents respiratory rate, with a unit of breaths per minute; VT represents tidal volume, $C_{rs}$ represents lung compliance, with a unit of ml/cmH2O; I:E represents a ratio of inhalation to exhalation (i.e. the ratio of inspiratory time to expiratory time); $R_{aw}$ represents airway resistance, with a unit of cmH2O/L/s; PEEP represents positive end expiratory pressure, with a unit of cmH2O; $PEEP_{volume}$ represents tidal volume caused by PEEP, with a unit of liter (L), and specifically represents the volume exhaled when PEEP drops to 0; Paw represents airway pressure, with a unit of cmH2O and can be measured using a preset pressure sensor of the respiratory support device; Flow represents the flow rate, with a unit of L/min, and can be obtained by using an external flow sensor at the patient end, or by monitoring a difference between the inspiratory flow sensor and the expiratory flow sensor of the respiratory support device; dt represents integration for time.

Specifically, the mechanical power corresponding to the ventilation of patient can be obtained by integrating the pressure (i.e., airway pressure) and flow rate (i.e., gas flow rate) within a single cycle, as follows:

$$Energy_{rs} = \int_0^{Tinsp} Paw \times Flow\ dt;$$

wherein, $Energy_{rs}$ represents the mechanical power which acts on the respiratory system of the patient by the ventilation and is obtained by integrating the airway pressure and gas flow rate within a single cycle; Tinsp represents the inspiratory time of each respiratory cycle, Paw represents airway pressure, and Flow represents gas flow rate. Of course, the mechanical power calculated for a single cycle can also be combined with respiratory rate to convert it into mechanical power per minute, as follows:

$$Power_{rs} = 0.098 \times RR \times \int_0^{Tinsp} Paw \times Flow\ dt;$$

wherein airway pressure Paw has a unit of cmH2O, inspiratory time of each respiratory cycle, Tinsp has a unit of seconds (s); RR represents respiratory rate, with a unit of each minute, the mechanical power $Power_{rs}$ which acts on the respiratory system of the patient by the ventilation and is obtained by integrating the airway pressure and gas flow rate has a unit of J/min. As $1cmH2O \times 1L/min=0.098J/min$, therefore there is a coefficient of 0.098 in the above formula. Of course, it is also possible to directly add up the mechanical power $Energy_{rs}$ of all cycles within one minute to obtain power per minute.

In some embodiments, when calculating the mechanical power of ventilation (i.e., mechanical ventilation) which acts on the respiratory system of the patient according to the airway pressure and gas flow rate, potential power generated by the tidal volume formed by positive end expiratory pressure can also be considered. This part of power is generally a fixed value, and does not change with mechanical ventilation, and it can often be omitted because additional positive end expiratory pressure release is required. When considering this part of potential power, the above formula can become:

$$Energy_{rs} = \int_0^{Tinsp} Paw \times Flow\ dt + \frac{1}{2} \times PEEP_{volume} \times PEEP.$$

The mechanical power obtained through combining with respiratory rate after conversion of units is as follows:

$$\text{Power}_{rs} = 0.098 \times \text{RR} \times \left( \int_0^{\text{Tinsp}} \text{Paw} \times \text{Flow dt} + \frac{1}{2} \times \text{PEEP}_{\text{volume}} \times \text{PEEP} \right) .$$

$\text{PEEP}_{\text{volume}}$ in both above formulas, represents tidal volume caused by positive end expiratory pressure, with a unit of L, which represents specifically the volume exhaled when the positive end expiratory pressure drop is 0, and PEEP is the positive end expiratory pressure.

In order to improve the accuracy and reliability of mechanical power determined, the respiratory support device can determine the mechanical power according to the corresponding respiratory rate, Tidal volume, inspiratory time, compliance, resistance, airway pressure and other ventilation parameters when the patient is ventilated. Compliance is the compliance of the lungs of the patient (also known as lung compliance).

Due to the impact of the set value for positive end expiratory pressure on collapse and excessive expansion of alveolar, positive end expiratory pressure can have a significant impact on lung compliance in patient. According to the respiratory mechanics model, the human respiratory system has characteristics such as resistance and compliance. When using the respiratory support device for mechanical ventilation, it is necessary to overcome the pressure generated by resistance (resistance caused by flow passing through the airway) and the pressure generated by compliance (pressure generated by alveolar inflation). Therefore, the respiratory support device needs to apply power on the respiratory system of the patient, and in order to maintain positive end expiratory pressure, the respiratory support device also needs to apply a certain amount of power to the respiratory system of the patient. The mechanical power during mechanical ventilation can be obtained by combining respiratory power and the power required to maintain positive end expiratory pressure. The formula can be as follows:

$$\text{Power}_{rs} = \text{RR} \times \text{VT} \times \left[ \left( \frac{\text{VT}}{2 \times C_{rs}} + \frac{\text{VT} \times \text{Raw}}{\text{Tinsp}} \right) + PEEP \right];$$

wherein, $\text{Power}_{rs}$ represents the mechanical power, with a unit of J/min; RR represents respiratory rate (such as the number of times the patient breathes in one minute); VT represents tidal volume (such as the amount of gas the patient inhales at one time); Tinsp represents inspiratory time, such as the inspiratory time of each respiratory cycle, with a unit of second(s), $C_{rs}$ represents the compliance of the lungs of the patient (characteristics of the lungs), Raw represents airway pressure (i.e. airway resistance), and PEEP represents positive end expiratory pressure.

In step 130, the ventilation parameter configuration is adjusted according to the mechanical power, so as to enable the mechanical power determined after the adjustment to satisfy a preset condition.

By automatically adjusting the mechanical ventilation parameter according to the monitoring result of mechanical power, it is possible to ensure the comfort and safety of clinical patient without the need for clinical adjustment of the doctor, automatically adjust the settings of mechanical ventilation parameter according to the guidance of mechanical power, thus reducing or avoiding the risk of ventilator-related lung injury for patient.

In the disclosure, adjusting the ventilation parameter configuration according to the mechanical power, so as to enable the mechanical power determined after the adjustment to satisfy a preset condition, includes: adjusting the ventilation parameter configuration according to the mechanical power, so as to enable the mechanical power determined after the adjustment to be less than or equal to a preset mechanical power threshold, and/or to be less than or equal to the mechanical power which is determined before the adjustment.

For example, by adjusting the ventilation parameter configuration, the mechanical power applied to the patient during ventilation is maintained at a lower level, such as less than or equal to the preset mechanical power threshold, or the mechanical power corresponding to the adjusted ventilation parameter configuration is less than or equal to the mechanical power determined before the adjustment, so that the mechanical power applied to the patient is reduced or stable, thus reducing or avoiding the risk of ventilator-related lung injury for the patient.

In the disclosure, adjusting the ventilation parameter configuration according to the mechanical power, so as to enable the mechanical power determined after the adjustment to satisfy a preset condition, includes: adjusting the ventilation parameter configuration according to the mechanical power, so as to enable the mechanical power determined after the adjustment to increase, compared to the mechanical power determined before the adjustment, wherein the increase is less than or equal to an increase threshold.

For example, the adjusted mechanical power can show a negative increase compared to the mechanical power determined before the adjustment, i.e., a decrease, or the adjusted mechanical power can remain unchanged compared to the mechanical power determined before the adjustment, or slightly increase, so as to result in a decrease or stability in the mechanical power applied to the patient, thus reducing or avoiding the risk of ventilator-related lung injury.

In the disclosure, adjusting the ventilation parameter configuration according to the mechanical power, so as to enable the mechanical power determined after the adjustment to satisfy a preset condition, includes: adjusting the ventilation parameter configuration according to the mechanical power, so as to minimize the mechanical power determined after the adjustment.

For example, when adjusting the ventilation parameter configuration, one ventilation parameter configuration can be determined from multiple ventilation parameter configurations, wherein the multiple ventilation parameter configurations can determine different mechanical power. By determining the ventilation parameter configuration with the minimum mechanical power as the adjusted ventilation parameter configuration, the mechanical power applied to the patient can be maintained at a lower level, thus reducing or avoiding the risk of ventilator-related lung injury for the patient. It can be understood that the concept of "minimum" in the minimum mechanical power determined after the adjustment is a relative conception, which specifically refers to the minimum mechanical power among various undetermined ventilation parameter configurations when adjusting the ventilation parameter configuration.

It should be noted that the mechanical power satisfies the preset condition, includes but is neither limited to the conditions listed above, nor limited to just one of the preset conditions. For example, the ventilation parameter configuration can be adjusted according to the mechanical power so that the mechanical power determined after the adjustment is either less than or equal to the preset mechanical power threshold, and has the increase compared to the mechanical power determined before the adjustment, which increase is less than or equal to the increase threshold.

In the disclosure, adjusting the ventilation parameter configuration according to the mechanical power, so as to enable the mechanical power determined after the adjustment to satisfy a preset condition, includes: determining multiple optional ventilation parameter configurations within a preset range of the set value for the ventilation parameter, which corresponds to the ventilation parameter configuration; determining mechanical power which corresponds to each optional ventilation parameter configuration; determining the optional ventilation parameter configuration, which corresponds to the mechanical power that satisfies the preset condition, as a target ventilation parameter configuration of the respiratory support device, so as to enable the respiratory support device to ventilate the patient according to the target ventilation parameter configuration.

For example, it is possible to determine the corresponding ventilation parameters, such as pressure and flow rate, when the respiratory support device ventilates the patient according to the optional ventilation parameter configuration, and obtain the mechanical power corresponding to each optional ventilation parameter configuration by integrating the pressure and flow rate. For example, the corresponding ventilation parameters, such as pressure and flow rate, during the ventilation of the patient in a predetermined time cycle, can be detected by the ventilation detection apparatus, when the respiratory support device ventilates the patient according to the multiple optional ventilation parameter configurations, such as positive end expiratory pressure (PEEP). Then the pressure and gas flow rate within the predetermined time cycle are integrated to obtain the mechanical power corresponding to each optional ventilation parameter configuration, and the mechanical power that satisfies the predetermined condition can be determined. Then the ventilation parameter configuration can be adjusted to enable it to be close to or reach the optional ventilation parameter configuration to which the mechanical power that satisfies the preset condition corresponds.

For example, the respiratory support device obtains multiple mechanical power which corresponds to different ventilation parameter configurations, according to the corresponding mechanical power when the respiratory support device ventilate the patient according to the different ventilation configurations, such as PEEP. For example, the respiratory support device obtains corresponding respiratory frequency, tidal volume, inspiratory time, compliance, airway resistance and airway pressure in a predetermined time cycle, when the respiratory support device ventilate the patient according to the different ventilation parameter configurations, such as PEEP; and determines the mechanical power according to the different ventilation parameter configurations, such as PEEP and their corresponding respiratory frequency, tidal volume, inspiratory time, compliance, airway resistance and airway pressure, so as to obtain the multiple mechanical power. The ventilation parameter configuration, which corresponds to the mechanical power that satisfies the preset condition, is determined as the target ventilation parameter configuration of the respiratory support device, so that the respiratory support device can ventilate the patient according to the target ventilation parameter configuration.

For example, determining multiple optional ventilation parameter configurations within a preset range of the set value for the ventilation parameter, which corresponds to the ventilation parameter configuration, includes determining the multiple optional ventilation parameter configurations within the preset range of the set value for the ventilation parameter, according to a preset step size.

For example, if the preset range of the set value for the ventilation parameter PEEP corresponding to the ventilation parameter configuration is 10-15cmH2O, the optional ventilation parameter configurations of 10cmH2O, 11cmH2O, 12cmH2O, 13cmH2O, 14cmH2O, and 14cmH2O can be determined, with the preset step size of 1cmH2O. By determining the corresponding ventilation parameters, such as pressure and flow rate, when the respiratory support device ventilates the patient according to each optional ventilation parameter configuration, and integrating the pressure and flow rate to obtain the mechanical power corresponding to each optional ventilation parameter configuration, the optional ventilation parameter configuration with the mechanical power that satisfies the preset condition, within the preset range of the set value for the at least one ventilation parameter, can be determined as the target ventilation parameter configuration of the respiratory support device. In this way, the respiratory support device ventilates the patient according to the target ventilation parameter configuration.

For example, different optional ventilation parameter configurations are be set as the ventilation parameter configuration at intervals to determine the corresponding mechanical power for each optional ventilation parameter configuration. For example, if the set value for the current ventilation parameter PEEP is 10cmH2O, the set value for the ventilation parameter PEEP is configured to be 11cmH2O at a preset interval to determine the mechanical power corresponding to the optional ventilation parameter configuration of 11cmH2O. After another preset time interval, the ventilation parameter PEEP is configured to be 12cmH2O to determine the mechanical power corresponding to the optional ventilation parameter configuration of 12cmH2O.

During the process in which the respiratory support device ventilates the patient according to different optional ventilation parameter configurations, optional ventilation parameters can be gradually increased or decreased, so as to find the optional ventilation parameter corresponding to the best mechanical power according to the changes of the mechanical power corresponding to different optional ventilation parameter configurations.

In the disclosure, the method further includes obtaining a designated monitoring parameter of the patient and/or a designated ventilation parameter of the respiratory support device.

For example, the designated monitoring parameter includes at least one of the following: end-expiratory carbon dioxide (ETCO2), drive pressure ($\Delta P$), ventilation volume per minute (MV), inspiratory plateau pressure (Pplat), and blood oxygen saturation (SPO2).

For example, the designated monitoring parameter of the patient can be obtained through at least one of the followings: the respiratory support device, and a monitor.

For example, the designated ventilation parameter includes a ventilation volume between the respiratory support device and the patient.

For example, adjusting the ventilation parameter configuration according to the mechanical power, so as to enable the mechanical power determined after the adjustment to satisfy a preset condition, includes:

adjusting the ventilation parameter configuration according to the mechanical power, the designated monitoring parameter and the designated ventilation parameter, so as to enable the mechanical power to satisfy the preset condition, when the designated monitoring parameter and the designated ventilation parameter are respectively within a corresponding preset safe range.

Understandably, when adjusting the ventilation parameter configuration according to the mechanical power, it is necessary to ensure the safety of the patient, and maintain the designated monitoring parameter of the patient and/or the designated ventilation parameter of the respiratory support device within the corresponding preset safe range. For example, the blood oxygen saturation of the patient is ensured to be within a safe range. Under the premise of ensuring the ventilation volume of the patient or maintaining the physiological indicators (such as oxygenation and CO2 levels) of the patient, the ventilation parameter can be automatically adjusted to ensure that the mechanical power satisfies the preset condition (such as minimum mechanical power), thus reducing lung injury.

For example, the ventilation parameter configuration is adjusted so that the mechanical power satisfies the preset condition, when the designated monitoring parameter and the designated ventilation parameter are within the corresponding preset safe range. For example, by setting the inhaled oxygen concentration (FiO2), the blood oxygen saturation can be maintained within a safe range of 88% -95%. If the blood oxygen saturation exceeds 95%, FiO2 can be reduced to a minimum of 21%. If the blood oxygen saturation is below 88%, FiO2 can be increased to a maximum of 100%.

In the disclosure, based on a preset initial ventilation parameter configuration, the ventilation parameter configuration is adjusted according to the mechanical power, the designated monitoring parameter, and the designated ventilation parameter; and/or within a preset adjustment range of the ventilation parameter configuration, the ventilation parameter configuration is adjusted according to the mechanical power, the designated monitoring parameter, and the designated ventilation parameter. In this way, the mechanical power satisfies the preset condition, when the designated monitoring parameter and the designated ventilation parameter are within the corresponding preset safe range.

For example, when adjusting the ventilation parameter configuration within the preset adjustment range, by setting a reasonable adjustment range of the ventilation parameter configuration, the mechanical power can satisfy the preset condition when the designated monitoring parameter and the designated ventilation parameter are respectively within the corresponding preset safe range. For example, the adjustment range for respiratory rate is set to 8-35 breaths/min, and the adjustment range for positive end expiratory pressure is set to 4-20cmH2O.

For example, the adjustment range can be determined according to the respiratory state of the patient, such as according to the compliance and/or airway resistance of the respiratory system of the patient. For example, when the compliance $C_{rs}$ of the respiratory system of the patient is greater than 50-70ml/cmH2O, it can be determined that the lung compliance of the patient is basically normal, when the compliance $C_{rs}$ of the respiratory system of the patient is less than 50ml/cmH2O, it can be determined that the patient has acute respiratory distress syndrome (ARDS). At least some values, in the adjustment range of positive end expiratory pressure, which range is determined when the compliance $C_{rs}$ is greater than 50-70ml/cmH2O, are less than values, in the adjustment range of positive end expiratory pressure, which range is determined when the compliance $C_{rs}$ is less than 50ml/cmH2O.

For example, by determining an appropriate initial ventilation parameter configuration, the mechanical power satisfies the preset condition when the designated monitoring parameter and the designated ventilation parameter are respectively within the corresponding preset safe range.

For example, the initial ventilation parameter configuration can be determined according to the respiratory state of the patient. In the disclosure, the method further includes determining the respiratory state of the patient at the beginning of ventilation, and determining the initial ventilation parameter configuration according to the respiratory state of the patient, so as to enable the respiratory support device to ventilate the patient according to the initial ventilation parameter configuration.

For example, determining the initial ventilation parameter configuration according to the respiratory state of the patient, includes: determining the compliance and/or airway resistance of the respiratory system of the patient; determining the initial ventilation parameter configuration according to at least one of the compliance and the airway resistance. For example, when the compliance $C_{rs}$ of the respiratory system of the patient is greater than 50-70ml/cmH2O, the initial positive end expiratory pressure can be determined to be 4-6 cmH2O; when the compliance $C_{rs}$ of the respiratory system of the patient is less than 50ml/cmH2O, the initial positive end expiratory pressure can be determined to be 5cmH2O, 10cmH2O, or 15cmH2O.

In the disclosure, the respiratory state of the patient may also include inhaled oxygen concentration FiO2 and/or blood oxygen saturation SPO2. For example, the adjustment range of the ventilation parameter configuration and/or the initial ventilation parameter configuration can be determined according to the inhaled oxygen concentration FiO2 and/or blood oxygen saturation SPO2. For example, when the compliance $C_{rs}$ of the respiratory system of the patient is less than 50ml/cmH2O, the initial positive end expiratory pressure and the adjustment range of the positive end expiratory pressure is set according to the range of SPO2/FiO2. Specifically, when SPO2/FiO2 is greater than 235 (equivalent to mild ARDS), the adjustment range of positive end expiratory pressure PEEP is 5-10cmH2O, and the initial positive end expiratory pressure is 5cmH2O. When 235>SPO2/FiO2>181 (equivalent to moderate ARDS), the adjustment range of positive end expiratory pressure PEEP is 10-15cmH2O, and the initial positive end expiratory pressure is 10cmH2O. When SPO2/FiO2 is less than 181 (equivalent to severe ARDS), the adjustment range of positive end expiratory pressure is 15-20cmH2O, and the initial positive end expiratory pressure is 15cmH2O.

In order to improve the convenience of the initial ventilation parameter configuration, the initial ventilation parameter configuration can be set according to the different conditions of patient. For example, severe respiratory diseases may require a higher value of positive end expiratory pressure. Specifically, a mapping relationship between different condition information of different patients and different initial ventilation parameter configurations can be preset. For example, a mapping relationship is established between patient identification (such as name or account number), different condition information and different initial ventilation parameter configurations. The mapping relationship can be the historical optimal value obtained during the historical adjustment of the respiratory support device, can also be pre-set by medical staff according to experience, and can also be generated through other methods. The specific content is not limited here.

For example, when the respiratory support device starts to ventilate the patient, the respiratory support device obtains the respiratory state of the patient and determines the initial ventilation parameter configuration according to the respiratory state, control the respiratory support device to ventilate the patient according to the initial ventilation parameter configuration, and obtains initial mechanical power corresponding to the respiratory support device after the respiratory support device ventilates the patient for a preset time according to the initial ventilation parameter configuration. Afterwards, real-time ventilation parameter configuration can be obtained according to a preset time interval and real-time mechanical power can be determined according to the ventilation parameter configuration. This time interval can be flexibly set according to actual requirements. If the initial mechanical power and/or the mechanical power do/does not satisfy the preset condition, the ventilation parameter configuration is adjusted. For example, multiple optional ventilation parameter configurations are determined within the adjustment range, the mechanical power corresponding to each optional ventilation parameter configuration is determined, and the optional ventilation parameter configuration corresponding to the mechanical power that satisfies the preset condition is determined as the target ventilation parameter configuration of the respiratory support device, so as to ventilate the patient according to the target ventilation parameter configuration, so that the mechanical power satisfies the preset condition.

In the disclosure, the method further includes normalizing the mechanical power, which is determined according to the ventilation parameter configuration. For example, adjusting the ventilation parameter configuration according to the mechanical power, includes: adjusting the ventilation parameter configuration according to the normalized mechanical power. By normalizing mechanical power, the amount of mechanical power can be standardized according to the information of individual patient, such as illness severity and/or physical fitness, etc. The ventilation parameter configuration adjusted by the mechanical power can be more in line with the patient.

For example, normalizing the mechanical power, which is determined according to the ventilation parameter configuration, includes: normalizing the mechanical power, which is determined according to the ventilation parameter configuration, according to at least one of body weight of the patient, vital capacity, ventilation volume of a current preset duration, compliance of respiratory system, and functional residual volume. For example, the normalized mechanical power can be a ratio of mechanical power to lung compliance $C_{rs}$, or a ratio of mechanical power to functional residual volume FRC, or a ratio of mechanical power to body weight of the patient.

The control method for the respiratory support device provided in this disclosure can determine the mechanical power according to the ventilation parameter configuration of the respiratory support device during ventilation of the patient, wherein the mechanical power is configured to indicate power of mechanical ventilation, which acts on lung(s) or a respiratory system; and adjust the ventilation parameter configuration according to the mechanical power, so as to enable the mechanical power determined after the adjustment to satisfy a preset condition. In this way, automatic adjustment of ventilation parameter configuration without the need for manual adjustment, is realized, the accuracy and timeliness of ventilation parameter configuration adjustment are improved, the risk of lung injury for patient is reduced or avoided, and the convenience and reliability of ventilation control for respiratory support device, are also improved.

In the disclosure, the control method for the respiratory support device provided in the embodiment of this disclosure includes the following steps.

1. Determine compliance (Crs) of respiratory system of the patient and/or airway resistance (Raw) of the patient.

Wherein, the compliance Crs can be determined by the following equation: Crs=VT÷ΔP=VT÷(Pplat-PEEP); wherein VT represents Tidal volume, ΔP represents drive pressure, Pplat represents inspiratory plateau pressure, and PEEP represents positive end expiratory pressure. Airway resistance can be determined by the following equation: Raw= (PIP Plat) ÷ Flow, wherein PIP represents peak airway pressure and Flow represents gas flow rate.

For example, after activating the automatic function of mechanical power oriented lung protective ventilation (or the function of automatic adjustment of ventilation parameter configuration, hereinafter referred to as the automatic function), the ventilator automatically adopts VCV mode, which is also called as volume-controlled mode in the ventilation mode. The tidal volume VT is stabilized at 6ml/PBW (preset kg body weight), the gas flow rate is stabilized at 30L/min, and the PEEP (positive end expiratory pressure) remains unchanged, the respiratory rate RR is increased to 25-30 breaths per minute to suppress spontaneous respiration. Meanwhile, the monitored end expiratory flow rate is 0 to avoid the generation of endogenous positive end expiratory pressure (PEEP). When the VCV mode reached a stable state, the tidal volume VT, inspiratory plateau pressure Pplat and positive end expiratory pressure PEEP at the stable state, are obtained to determine the compliance Crs of the respiratory system of the patient, and the tidal volume VT, inspiratory plateau pressure Pplat and the gas flow rate Flow at the stable state, are obtained to determine the airway resistance Raw of the patient.

2. Determine an initial ventilation parameter configuration and/or an adjustment range of a ventilation parameter configuration.

(i). Determine and adjust inhaled oxygen concentration FiO2. The determination and adjustment of inhaled oxygen concentration FiO2 ensure that the blood oxygen saturation SPO2 is within the safe range of 88% -95%. If the blood oxygen saturation exceeds 95%, the FiO2 can be reduced to a minimum of 21%. If the blood oxygen saturation is below 88%, the FiO2 can be increased to a maximum of 100%.

(ii). Determine and adjust positive end expiratory pressure PEEP. When the compliance $C_{rs}$ of the respiratory system of the patient is greater than 50-70ml/cmH2O, it can be determined that the lung compliance of the patient is basically normal, the initial positive end expiratory pressure can be determined to be 4-6 cmH2O. When the compliance $C_{rs}$ of the respiratory system of the patient is less than 50ml/cmH2O, it can be determined that the patient has acute respiratory distress syndrome (ARDS), the initial positive end expiratory pressure PEEP and the adjustment range of positive end expiratory pressure is set according to the range of SPO2/FiO2. Specifically, when SPO2/FiO2 is greater than 235 (equivalent to mild ARDS), the adjustment range of positive end expiratory pressure PEEP is 5-10cmH2O, and the initial positive end expiratory pressure is 5cmH2O. When 235>SPO2/FiO2>181 (equivalent to moderate ARDS), the adjustment range of positive end expiratory pressure is 10-15cmH2O, and the initial positive end expiratory pressure is 10cmH2O. When SPO2/FiO2 is less than 181 (equivalent to severe ARDS), the adjustment range of positive end expiratory pressure is 15-20cmH2O, and the initial positive end expiratory pressure is 15cmH2O. The positive end expiratory pressure (PEEP) is adjusted to increase by 1cmH2O every 3 minutes with the ventilation parameter configuration other than PEEP unchanged, so as to enable the mechanical power monitored within the PEEP setting range to satisfy the preset condition, such as reaching the minimum.

(iii). Set and adjust respiratory rate RR. The initial respiratory rate RR is set to original respiratory rate of the patient or 16 breaths/min, and the adjustment range of respiratory rate is between 8-35 breaths/min. Under the condition that the ventilation parameters, such as Tidal volume VT, are constant, the respiratory rate RR is adjusted according to the principle of minimum mechanical power. The goal of adjustment is that the end expiratory carbon dioxide ETCO2 is between 30-50mmHg, and the ventilation volume per minute MV is not less than 5/min. If ETCO2 is greater than 40mmHg, the respiratory rate is increased by a step size of 2 breaths/min. If ETCO2 is less than 40mmHg, the Tidal volume VT or inspiratory pressure Pinsp is adjusted firstly, and meanwhile the mechanical power is monitored to enable the mechanical power to reach the minimum value. The respiratory rate RR is adjusted after adjusting the Tidal volume VT or inspiratory pressure Pinsp. The inspiratory pressure Pinsp is configured to indicate the target value of ventilation pressure, such as the target value of airway pressure Paw.

(iv). Determine the initial ventilation parameter configuration. If the ventilator adopts VCV mode, which is also called as volume-controlled mode in the ventilation mode. The tidal volume VT is stabilized at 6ml/PBW (preset kg body weight), and the drive pressure $\Delta P$ and inspiratory plateau pressure Pplat are monitored. Take $\Delta P < 15cmH2O$ and Pplat<30cmH2O as the safety range for VT settings. If the safety range is exceeded, VT is reduced to the safety range in a step size of 0.5ml/PBW. It is determined that the end expiratory carbon dioxide (ETCO2) is between 30-50mmHg. If ETCO2 is less than 40mmHg, VT is reduced by a step size of 0.5ml/PBW. When adjusting VT and RR, the mechanical power is monitored, so as to enable the mechanical power to reach the minimum value within the safe range. If the ventilator adopts VCV mode, the initial gas flow rate Flow is set to 30L/min, and the mechanical power is monitored to determine the safe range of inspiratory time Ti is from 0.6 to 1.4 seconds. With the ventilation parameters other than gas flow rate unchanged, the gas flow rate Flow is gradually adjusted in a step size of 5L/min, so as to enable the mechanical power to reach the minimum value within the safe range. If the ventilator adopts PCV mode, which is also called as pressure-controlled mode, the inspiratory pressure Pinsp required for the initial setting of 6ml/PBW for the Tidal volume is calculated according to the compliance (Crs) of the respiratory system of the patient and the airway resistance (Raw) of the patient. The safety range of the inspiratory time Ti is 0.6-1.4s. The inspiratory time Ti is adjusted to enable the end inspiratory flow rate to be 0. The drive pressure $\Delta P$ and inspiratory plateau pressure Pplat are monitored. Take $\Delta P < 15cmH2O$ and Pplat<30cmH2O as the safety range for inspiratory pressure Pinsp setting. If the safety range is exceeded, inspiratory pressure Pinsp is reduced to the safety range in a step size of 1cmH2O. The end expiratory carbon dioxide ETCO2 is between 30-50mmHg. If ETCO2<40mmHg, the inspiratory pressure Pisnp is reduced by a step size of 1cmH2O. When adjusting inspiratory pressure Pisnp and respiratory rate RR, the mechanical power is monitored, so as to enable the mechanical power to reach the minimum value within the safe range. If the ventilator adopts PCV mode, which is also called as pressure-controlled mode, the pressure rise time Ramp is adjusted after the inspiratory pressure Pisnp is set. The range of pressure rise time Ramp is 0-0.3 seconds, with an initial setting of 0.05 seconds. If there is pressure overshoot, the pressure rise time Ramp is reduced by a step size of 0.05, while the mechanical power is monitored, so as to enable the mechanical power to reach the minimum value within the safe range.

By automatically adjusting the ventilation parameter configuration mentioned above, the mechanical ventilation parameter configuration can be optimized according to mechanical power while ensuring ventilation safety of patient, so as to alleviate lung injury (VILI).

Please refer to FIG. 2. FIG. 2 is a structural diagram for a respiratory support device provided in an embodiment of this disclosure;

As shown in FIG. 2, the respiratory support device includes an gas flow supply apparatus 10 and a respiration pipeline 20, which is connected with the gas flow supply apparatus 10 to transmit ventilation gas flow generated by the gas flow supply apparatus 10 to an airway of a patient.

For example, the respiratory support device further includes a patient interface 30, which includes a mask, a nasal mask, a nasal cannula, a tracheal intubation, and the likes, which are attached to the patient. Wherein, the gas flow supply apparatus 10 is connected to the patient interface 30 through the respiratory line 20, for transmitting the ventilation gas flow to the airway of the patient.

For example, the respiratory support device further includes a ventilation detection apparatus 40, which is arranged at the respiratory line or patient interface to detect ventilation parameters, which include a flow rate of the ventilation gas flow, an airway pressure, a respiratory frequency, a tidal volume, inspiratory time, respiratory system compliance or lung compliance, etc. It should be noted that the ventilation parameters can be either directly detected or calculated after detecting certain basic parameters.

For example, some ventilation parameters, such as ventilation volume, can also be obtained through devices other than respiratory support devices, such as monitors.

Specifically, the respiratory support device includes one or more processors 50, which work individually or collectively to execute the steps of the control method for the respiratory support device. The processor 50 can be a Central Processing Unit (CPU), other general-purpose processor, Digital Signal Processor (DSP), Application Designated Integrated Circuit (ASIC), Field Programmable Gate Array (FPGA), or other programmable logic device, discrete gate, or transistor logic devices, discrete hardware component, etc. Wherein, the general-purpose processor can be a microprocessor or any other conventional processor.

For example, the respiratory support device further includes a memory 60, and the processor 50 and the memory 60 can be connected through a bus, such as I2C (Inter integrated Circuit) bus.

The memory 60 may be volatile memory, such as random-access memory (RAM), or non-volatile memory, such as read-only memory (ROM), flash memory, hard disk drive (HDD) or solid-state drive (SSD); or a combination of the above types of memories. The memory 60 is configured to store computer programs and can provide instructions and data to processor 50.

Wherein, the processor 50 is configured to execute a computer program stored in the memory 60, and to implement any control method for respiratory support device provided in an embodiment of this disclosure when executing the computer program.

The respiratory support device further includes a human-machine interaction apparatus, which includes a display for displaying positive end expiratory pressure when the respiratory support device ventilates the patient, as well as displaying patient state information, ventilation parameters, etc. The specific display content includes text, chart, number, color, waveform, character, etc., for intuitively displaying various types of information. In practical applications, the human-machine interaction apparatus further includes an input device, through which medical staff can set various parameters, as well as select and control a display interface of the display, to achieve information exchange between human and machine. This display can also be a touch display.

In some embodiments, the respiratory support device may be a ventilator or an anesthesia machine.

Specifically, the one or more processors 50 work individually or collectively to perform the following steps: obtaining a ventilation parameter configuration of the respiratory support device during ventilation for a patient, wherein the ventilation parameter configuration includes a set value for at least one ventilation parameter; determining mechanical power according to the ventilation parameter configuration, wherein the mechanical power is configured to indicate power of mechanical ventilation, which acts on lung(s) or a respiratory system; adjusting the ventilation parameter configuration according to the mechanical power, so as to enable the mechanical power determined after the adjustment to satisfy a preset condition.

In some embodiments, when the one or more processors perform the step of adjusting the ventilation parameter configuration according to the mechanical power, so as to enable the mechanical power determined after the adjustment to satisfy a preset condition, the one or more processors specifically perform following step: adjusting the ventilation parameter configuration according to the mechanical power, so as to enable the mechanical power determined after the adjustment to be less than or equal to a preset mechanical power threshold, and/or to be less than or equal to the mechanical power which is determined before the adjustment.

In some embodiments, when the one or more processors perform the step of adjusting the ventilation parameter configuration according to the mechanical power, so as to enable the mechanical power determined after the adjustment to satisfy a preset condition, the one or more processors specifically perform following step: adjusting the ventilation parameter configuration according to the mechanical power, so as to enable the mechanical power determined after the adjustment to increase, compared to the mechanical power determined before the adjustment, wherein the increase is less than or equal to an increase threshold.

In some embodiments, when the one or more processors perform the step of adjusting the ventilation parameter configuration according to the mechanical power, so as to enable the mechanical power determined after the adjustment to satisfy a preset condition, the one or more processors specifically perform following step: adjusting the ventilation parameter configuration according to the mechanical power, so as to minimize the mechanical power determined after the adjustment.

In some embodiments, when the one or more processors perform the step of adjusting the ventilation parameter configuration according to the mechanical power, so as to enable the mechanical power determined after the adjustment to satisfy a preset condition, the one or more processors specifically perform following steps:
determining multiple optional ventilation parameter configurations within a preset range of the set value for the at least one ventilation parameter, which corresponds to the ventilation parameter configuration; determining mechanical power which corresponds to each optional ventilation parameter configuration; determining the optional ventilation parameter configuration, which corresponds to the mechanical power that satisfies the preset condition, as a target ventilation parameter configuration of the respiratory support device, so as to enable the respiratory support device to ventilate the patient according to the target ventilation parameter configuration.

In some embodiments, the one or more processors further perform following step: obtaining a designated monitoring parameter of the patient and/or a designated ventilation parameter of the respiratory support device.

For example, when the one or more processors perform the step of adjusting the ventilation parameter configuration according to the mechanical power, so as to enable the mechanical power determined after the adjustment to satisfy a preset condition, the one or more processors specifically perform following steps:
adjusting the ventilation parameter configuration according to the mechanical power, the designated monitoring parameter and the designated ventilation parameter, so as to enable the mechanical power to satisfy the preset condition, when the designated monitoring parameter and the designated ventilation parameter are respectively within a corresponding preset safe range.

For example, when the one or more processors perform the step of adjusting the ventilation parameter configuration according to the mechanical power, the designated monitoring parameter and the designated ventilation parameter, the one or more processors specifically perform following steps:

adjusting, based on a preset initial ventilation parameter configuration, the ventilation parameter configuration, according to the mechanical power, the designated monitoring parameter, and the designated ventilation parameter; and/or

adjusting, within a preset adjustment range of the ventilation parameter configuration, the ventilation parameter configuration, according to the mechanical power, the designated monitoring parameter, and the designated ventilation parameter.

For example, when the one or more processors perform the step of obtaining a designated monitoring parameter of the patient, the one or more processors specifically perform following step:
obtaining the designated monitoring parameter of the patient, through at least one of: the respiratory support device and a monitor.

In some embodiments, the one or more processors further perform following steps:

determining a respiratory state of the patient, when the respiratory support device starts to ventilate the patient; determining an initial ventilation parameter configuration according to the respiratory state, so as to enable the respiratory support device to ventilate the patient according to the initial ventilation parameter configuration.

For example, when the one or more processors perform the step of determining an initial ventilation parameter configuration according to the respiratory state, the one or more processors specifically perform following steps:
determining compliance and/or airway resistance of the respiratory system of the patient; determining the initial ventilation parameter configuration according to at least one of the compliance and the airway resistance.

In some embodiments, the one or more processors further perform following step: normalizing the mechanical power, which is determined according to the ventilation parameter configuration.

For example, when the one or more processors perform the step of adjusting the ventilation parameter configuration according to the mechanical power, the one or more processors specifically perform following step:
adjusting the ventilation parameter configuration according to the normalized mechanical power.

For example, when the one or more processors perform the step of normalizing the mechanical power, which is determined according to the ventilation parameter configuration, the one or more processors specifically perform following step:
normalizing the mechanical power, which is determined according to the ventilation parameter configuration, according to at least one of body weight, vital capacity, ventilation volume of a current preset duration, compliance of respiratory system, and functional residual volume, of the patient.

The designated principle and implementation method of the respiratory support device provided in the embodiment of this disclosure are similar to the control method for the respiratory support device, and is not repeated here.

The embodiment not being part of this invention also provides a computer readable storage medium, which stores a computer program. When the computer program is executed by the processor, the steps of the control method for the respiratory support device provided in the above embodiment can be implemented.

Wherein, the computer-readable storage medium can be an internal storage unit of the respiratory support device described in any of the aforementioned embodiments, such as a hard disk or memory of the respiratory support device. The computer-readable storage medium can also be an external storage device of the respiratory support device, such as a plug-in hard disk, smart media card (SMC), secure digital (SD) card, flash card, etc., provided on the respiratory support device.

In an embodiment not being part of this invention, a computer program is also provided, which includes program instructions. When the program instruction is executed by the processor, the steps of the control method for the respiratory support device provided in the above embodiment can be implemented.

The respiratory support device and computer-readable storage medium of this disclosure determine mechanical power according to the ventilation parameter configuration of the respiratory support device during ventilation of the patient, wherein the mechanical power is configured to indicate power of mechanical ventilation, which acts on lung(s) or a respiratory system; and adjust the ventilation parameter configuration according to the mechanical power, so as to enable the mechanical power determined after the adjustment to satisfy a preset condition. In this way, automatic adjustment of ventilation parameter configuration without the need for manual adjustment, is realized, the accuracy and timeliness of ventilation parameter configuration adjustment are improved, the risk of lung injury for patient is reduced or avoided, and the convenience and reliability of ventilation control for respiratory support device, are also improved.

It should be understood that the terms used in this disclosure are only for the purpose of describing specific embodiments and are not intended to limit this disclosure.

It should also be understood that the term "and/or" used in this disclosure and the accompanying claims refers to any combination and all possible combinations of one or more of the related listed items, and includes these combinations.
The above are only the specific embodiments of this disclosure, and the protection scope of this disclosure is limited by the claims.

**Claims**

1.  A respiratory support device, comprising:

    a gas flow supply apparatus (10), which is configured to generate a ventilation gas flow;
    a respiratory line (20), which is connected with the gas flow supply apparatus (10) and configured to transmit the ventilation gas flow, which is generated by the gas flow supply apparatus (10), to an airway of a patient; and
    one or more processors (50), which work individually or collectively, so as to perform following steps:

        obtaining a ventilation parameter configuration of the respiratory support device during mechanical ventilation for a patient, wherein the ventilation parameter configuration comprises a set value for at least one ventilation parameter; **characterized by**
        determining mechanical power according to the ventilation parameter configuration, wherein the mechanical power is configured to indicate power of the mechanical ventilation, which acts on lung(s) or a respiratory system; and
        adjusting the ventilation parameter configuration according to the mechanical power, so as to enable the mechanical power determined after the adjustment to satisfy a preset condition.

2.  The respiratory support device according to claim **1, characterized in that**, when the one or more processors (50) perform the step of adjusting the ventilation parameter configuration according to the mechanical power, so as to enable the mechanical power determined after the adjustment to satisfy the preset condition, the one or more processors (50) specifically perform following step:
    adjusting the ventilation parameter configuration according to the mechanical power, so as to enable the mechanical power determined after the adjustment to be less than or equal to a preset mechanical power threshold, and/or to be less than or equal to the mechanical power which is determined before the adjustment.

3.  The respiratory support device according to claim 1, **characterized in that**, when the one or more processors (50) perform the step of adjusting the ventilation parameter configuration according to the mechanical power, so as to enable the mechanical power determined after the adjustment to satisfy the preset condition, the one or more processors specifically perform following step:
    adjusting the ventilation parameter configuration according to the mechanical power, so as to enable the mechanical power determined after the adjustment to increase, compared to the mechanical power determined before the adjustment, wherein said increase is less than or equal to an increase threshold.

4.  The respiratory support device according to claim 1, **characterized in that**, when the one or more processors (50) perform the step of adjusting the ventilation parameter configuration according to the mechanical power, so as to enable the mechanical power determined after the adjustment to satisfy the preset condition, the one or more processors (50) specifically perform following step:
    adjusting the ventilation parameter configuration according to the mechanical power, so as to minimize the mechanical power determined after the adjustment.

5.  The respiratory support device according to any one of claims 1-4, **characterized in that**, when the one or more processors (50) perform the step of adjusting the ventilation parameter configuration according to the mechanical power, so as to enable the mechanical power determined after the adjustment to satisfy the preset condition, the one or more processors (50) specifically perform following steps:

    determining multiple optional ventilation parameter configurations within a preset range of the set value for the at least one ventilation parameter, wherein the at least one ventilation parameter corresponds to the ventilation parameter configuration;
    determining the mechanical power which corresponds to each optional ventilation parameter configuration; and
    determining an optional ventilation parameter configuration, which corresponds to the mechanical power that satisfies the preset condition, as a target ventilation parameter configuration of the respiratory support device, so as to enable the respiratory support device to ventilate the patient according to the target ventilation parameter configuration.

6.  The respiratory support device according to any one of claims 1-4, **characterized in that**, the one or more processors (50) further perform following step:

    obtaining a designated monitoring parameter of the patient and/or a designated ventilation parameter of the respiratory support device;
    when the one or more processors (50) perform the step of adjusting the ventilation parameter configuration according to the mechanical power, so as to enable the mechanical power determined after the adjustment to satisfy a preset condition, the one or more processors (50) specifically perform following step:
    adjusting the ventilation parameter configuration according to the mechanical power, the designated monitoring parameter and the designated ventilation parameter, so as to enable the mechanical power determined after the adjustment to satisfy the preset condition, when the designated monitoring parameter and the designated ventilation parameter are respectively within a corresponding preset safe range.

7.  The respiratory support device according to claim 6, **characterized in that**, when the one or more processors (50) perform the step of adjusting the ventilation parameter configuration according to the mechanical power, the designated monitoring parameter and the designated ventilation parameter, the one or more processors (50) specifically perform following steps:

    adjusting, based on a preset initial ventilation parameter configuration, the ventilation parameter configuration, according to the mechanical power, the designated monitoring parameter, and the designated ventilation parameter; and/or
    adjusting, within a preset adjustment range of the ventilation parameter configuration, the ventilation parameter configuration, according to the mechanical power, the designated monitoring parameter, and the designated ventilation parameter.

8.  The respiratory support device according to claim 6, **characterized in that**, when the one or more processors (50) perform the step of obtaining the designated monitoring parameter of the patient, the one or more processors (50) specifically perform following step:
    obtaining the designated monitoring parameter of the patient, through at least one of: the respiratory support device and a monitor.

9.  The respiratory support device according to any one of claims 1-4, **characterized in that**, the one or more processors (50) further perform following steps:

    determining a respiratory state of the patient, when the respiratory support device starts to ventilate the patient; and
    determining an initial ventilation parameter configuration according to the respiratory state, so as to enable the respiratory support device to ventilate the patient according to the initial ventilation parameter configuration.

10. The respiratory support device according to claim 9, **characterized in that**, when the one or more processors (50) perform the step of determining an initial ventilation parameter configuration according to the respiratory state, the one or more processors (50) further perform following steps:

    determining compliance and/or airway resistance of the respiratory system of the patient; and
    determining the initial ventilation parameter configuration according to at least one of the compliance and the airway resistance.

11. The respiratory support device according to any one of claims 1-4, **characterized in that**, the one or more processors (50) further perform following step:

    normalizing the mechanical power, which is determined according to the ventilation parameter configuration;
    wherein when the one or more processors (50) perform the step of adjusting the ventilation parameter configuration according to the mechanical power, the one or more processors (50) further perform following step:
    adjusting the ventilation parameter configuration according to the normalized mechanical power.

12. The respiratory support device according to claim 11, **characterized in that**, when the one or more processors (50) perform the step of normalizing the mechanical power, which is determined according to the ventilation parameter configuration, the one or more processors (50) further perform following step:

normalizing the mechanical power, which is determined according to the ventilation parameter configuration, according to at least one of body weight, vital capacity, ventilation volume of a current preset duration, compliance of the respiratory system, and functional residual volume, of the patient.

**Patentansprüche**

1. Eine Atemunterstützungsvorrichtung, Folgendes umfassend:

eine Gasfluss-Versorgungsvorrichtung (10), die so ausgelegt ist, dass sie einen Beatmungsgasfluss erzeugt; eine Beatmungsleitung (20), die mit der Gasfluss-Versorgungsvorrichtung (10) verbunden ist und so ausgelegt ist, dass sie den von der Gasfluss-Versorgungsvorrichtung (10) erzeugten Beatmungsgasfluss zu den Atemwegen eines Patienten leitet; und einen oder mehrere Prozessoren (50), die einzeln oder gemeinsam arbeiten, um die folgenden Schritte auszuführen:

das Erhalten einer Beatmungsparameterkonfiguration der Atemunterstützungsvorrichtung während der mechanischen Beatmung eines Patienten, wobei die Beatmungsparameterkonfiguration einen Sollwert für mindestens einen Beatmungsparameter umfasst; **gekennzeichnet durch** das Bestimmen der mechanischen Leistung gemäß der Beatmungsparameterkonfiguration, wobei die mechanische Leistung so ausgelegt ist, dass sie die Leistung der mechanischen Beatmung angibt, die auf die Lunge(n) oder das Atmungssystem wirkt; und das Einstellen der Beatmungsparameterkonfiguration gemäß der mechanischen Leistung, sodass die nach der Einstellung bestimmte mechanische Leistung eine voreingestellte Bedingung erfüllt.

2. Die Atemunterstützungsvorrichtung nach Anspruch 1, **dadurch gekennzeichnet, dass**, wenn der eine oder die mehreren Prozessoren (50) den Schritt des Einstellens der Beatmungsparameterkonfiguration entsprechend der mechanischen Leistung ausführen, um zu ermöglichen, dass die nach der Einstellung bestimmte mechanische Leistung die voreingestellte Bedingung erfüllt, der eine oder die mehreren Prozessoren (50) speziell den folgenden Schritt ausführen:
das Einstellen der Beatmungsparameterkonfiguration entsprechend der mechanischen Leistung, sodass die nach der Einstellung bestimmte mechanische Leistung kleiner oder gleich einem voreingestellten Schwellenwert für die mechanische Leistung und/oder kleiner oder gleich der vor der Einstellung bestimmten mechanischen Leistung ist.

3. Die Atemunterstützungsvorrichtung nach Anspruch 1, **dadurch gekennzeichnet, dass**, wenn der eine oder die mehreren Prozessoren (50) den Schritt des Einstellens der Beatmungsparameterkonfiguration entsprechend der mechanischen Leistung ausführen, um zu ermöglichen, dass die nach der Einstellung bestimmte mechanische Leistung die voreingestellte Bedingung erfüllt, der eine oder die mehreren Prozessoren speziell den folgenden Schritt ausführen:
die Einstellung der Beatmungsparameterkonfiguration entsprechend der mechanischen Leistung, sodass die nach der Einstellung bestimmte mechanische Leistung im Vergleich zu der vor der Einstellung bestimmten mechanischen Leistung erhöht wird, wobei die Erhöhung kleiner oder gleich einem Erhöhungsschwellenwert ist.

4. Die Atemunterstützungsvorrichtung nach Anspruch 1, **dadurch gekennzeichnet, dass**, wenn der eine oder die mehreren Prozessoren (50) den Schritt des Einstellens der Beatmungsparameterkonfiguration entsprechend der mechanischen Leistung ausführen, um zu ermöglichen, dass die nach der Einstellung bestimmte mechanische Leistung die voreingestellte Bedingung erfüllt, der eine oder die mehreren Prozessoren (50) speziell den folgenden Schritt ausführen:
das Einstellen der Beatmungsparameterkonfiguration entsprechend der mechanischen Leistung, um die nach der Einstellung ermittelte mechanische Leistung zu minimieren.

5. Die Atemunterstützungsvorrichtung nach einem der Ansprüche 1 bis 4, **dadurch gekennzeichnet, dass**, wenn der eine oder die mehreren Prozessoren (50) den Schritt des Einstellens der Beatmungsparameterkonfiguration entsprechend der mechanischen Leistung ausführen, um zu ermöglichen, dass die nach der Einstellung bestimmte mechanische Leistung die voreingestellte Bedingung erfüllt, der eine oder die mehreren Prozessoren (50) speziell die folgenden Schritte ausführen:

das Bestimmen mehrerer optionaler Beatmungsparameterkonfigurationen innerhalb eines voreingestellten Bereichs des Einstellwerts für den mindestens einen Beatmungsparameter, wobei der mindestens eine Beatmungsparameter der Beatmungsparameterkonfiguration entspricht;
das Bestimmen der mechanischen Leistung, die jeder optionalen Beatmungsparameterkonfiguration entspricht; und
das Bestimmen einer optionalen Beatmungsparameterkonfiguration, die der mechanischen Leistung entspricht, die die vorgegebene Bedingung erfüllt, zur Zielbeatmungsparameterkonfiguration der Atemunterstützungsvorrichtung, um es der Atemunterstützungsvorrichtung zu ermöglichen, den Patienten gemäß der Zielbeatmungsparameterkonfiguration zu beatmen.

6. Die Atemunterstützungsvorrichtung nach einem der Ansprüche 1 bis 4, **dadurch gekennzeichnet, dass** der eine oder die mehreren Prozessoren (50) ferner den folgenden Schritt ausführen:

das Erfassen eines bestimmten Überwachungsparameters des Patienten und/oder eines bestimmten Beatmungsparameters der Atemunterstützungsvorrichtung;
wenn der eine oder die mehreren Prozessoren (50) den Schritt des Einstellens der Beatmungsparameterkonfiguration entsprechend der mechanischen Leistung ausführen, um zu ermöglichen, dass die nach der Einstellung bestimmte mechanische Leistung eine voreingestellte Bedingung erfüllt, führen der eine oder die mehreren Prozessoren (50) speziell den folgenden Schritt aus:
das Einstellen der Beatmungsparameterkonfiguration entsprechend der mechanischen Leistung, dem festgelegten Überwachungsparameter und dem festgelegten Beatmungsparameter, damit die nach der Einstellung bestimmte mechanische Leistung die voreingestellte Bedingung erfüllt, wenn der festgelegte Überwachungsparameter und der festgelegte Beatmungsparameter jeweils innerhalb eines entsprechenden voreingestellten Sicherheitsbereichs liegen.

7. Die Atemunterstützungsvorrichtung nach Anspruch 6, **dadurch gekennzeichnet, dass**, wenn der eine oder die mehreren Prozessoren (50) den Schritt des Einstellens der Beatmungsparameterkonfiguration entsprechend der mechanischen Leistung, dem festgelegten Überwachungsparameter und dem festgelegten Beatmungsparameter ausführen, der eine oder die mehreren Prozessoren (50) speziell die folgenden Schritte ausführen:

das Einstellen der Beatmungsparameterkonfiguration auf der Grundlage einer voreingestellten anfänglichen Beatmungsparameterkonfiguration entsprechend der mechanischen Leistung, dem festgelegten Überwachungsparameter und dem festgelegten Beatmungsparameter; und/oder
das Einstellen der Beatmungsparameterkonfiguration innerhalb eines voreingestellten Einstellungsbereichs der Beatmungsparameterkonfiguration entsprechend der mechanischen Leistung, dem festgelegten Überwachungsparameter und dem festgelegten Beatmungsparameter.

8. Die Atemunterstützungsvorrichtung nach Anspruch 6, **dadurch gekennzeichnet, dass**, wenn der eine oder die mehreren Prozessoren (50) den Schritt des Erhaltens des festgelegten Überwachungsparameters des Patienten ausführen, der eine oder die mehreren Prozessoren (50) speziell den folgenden Schritt ausführen:
das Erfassen des festgelegten Überwachungsparameters des Patienten durch mindestens eines der folgenden Mittel: die Atemunterstützungsvorrichtung oder einen Monitor.

9. Die Atemunterstützungsvorrichtung nach einem der Ansprüche 1 bis 4, **dadurch gekennzeichnet, dass** der eine oder die mehreren Prozessoren (50) ferner die folgenden Schritte ausführen:

das Bestimmen eines Atemzustands des Patienten, wenn die Atemunterstützungsvorrichtung beginnt, den Patienten zu beatmen; und
das Bestimmen einer anfänglichen Beatmungsparameterkonfiguration entsprechend dem Atemzustand, um es der Atemunterstützungsvorrichtung zu ermöglichen, den Patienten entsprechend der anfänglichen Beatmungsparameterkonfiguration zu beatmen.

10. Die Atemunterstützungsvorrichtung nach Anspruch 9, **dadurch gekennzeichnet, dass**, wenn der eine oder die mehreren Prozessoren (50) den Schritt des Bestimmens einer anfänglichen Beatmungsparameterkonfiguration entsprechend dem Atemzustand ausführen, der eine oder die mehreren Prozessoren (50) ferner die folgenden Schritte ausführen:

das Bestimmen der Lungendehnbarkeit und/oder des Atemwegswiderstands des Atmungssystems des Patienten; und

das Bestimmen der anfänglichen Beatmungsparameterkonfiguration entsprechend mindestens einem der Werte Lungendehnbarkeit oder Atemwegswiderstand.

**11.** Die Atemunterstützungsvorrichtung nach einem der Ansprüche 1 bis 4, **dadurch gekennzeichnet, dass** der eine oder die mehreren Prozessoren (50) ferner folgenden Schritt ausführen:

das Normalisieren der mechanischen Leistung, die gemäß der Beatmungsparameterkonfiguration bestimmt wird;
wobei, wenn der eine oder die mehreren Prozessoren (50) den Schritt des Einstellens der Beatmungsparameterkonfiguration gemäß der mechanischen Leistung ausführen, der eine oder die mehreren Prozessoren (50) ferner den folgenden Schritt ausführen:
das Einstellen der Beatmungsparameterkonfiguration entsprechend der normalisierten mechanischen Leistung.

**12.** Die Atemunterstützungsvorrichtung nach Anspruch 11, **dadurch gekennzeichnet, dass**, wenn der eine oder die mehreren Prozessoren (50) den Schritt der Normalisierung der mechanischen Leistung ausführen, die gemäß der Beatmungsparameterkonfiguration bestimmt wird, der eine oder die mehreren Prozessoren (50) ferner den folgenden Schritt ausführen:
das Normalisieren der mechanischen Leistung, die gemäß der Beatmungsparameterkonfiguration bestimmt wird, gemäß mindestens einem der folgenden Werte: Körpergewicht, Vitalkapazität, Beatmungsvolumen einer aktuell voreingestellten Dauer, Lungendehnbarkeit des Atmungssystems und funktionelles Residualvolumen des Patienten.

**Revendications**

**1.** Dispositif d'assistance respiratoire, comprenant :

un appareil de fourniture d'écoulement gazeux (10), lequel est configuré pour générer un écoulement gazeux de ventilation ;
une ligne respiratoire (20), laquelle est reliée à l'appareil de fourniture d'écoulement gazeux (10) et configurée pour transmettre l'écoulement gazeux de ventilation, qui est généré par l'appareil de fourniture d'écoulement gazeux (10), à une voie respiratoire d'un patient ; et
un ou plusieurs processeurs (50), lesquels fonctionnent individuellement ou collectivement, de sorte à réaliser les étapes suivantes :

obtenir une configuration de paramètre de ventilation du dispositif d'assistance respiratoire durant une ventilation mécanique d'un patient, où la configuration de paramètre de ventilation comprend une valeur de consigne d'au moins un paramètre de ventilation ; **caractérisé par**
déterminer une puissance mécanique selon la configuration de paramètre de ventilation, où la puissance mécanique est configurée pour indiquer une puissance de la ventilation mécanique, qui agit sur un ou les poumons ou un système respiratoire ; et
ajuster la configuration de paramètre de ventilation selon la puissance mécanique, de sorte à permettre à la puissance mécanique déterminée après l'ajustement de satisfaire à une condition préétablie.

**2.** Dispositif d'assistance respiratoire selon la revendication 1, **caractérisé en ce que**, lorsque les un ou plusieurs processeurs (50) réalisent l'étape consistant à ajuster la configuration de paramètre de ventilation selon la puissance mécanique, de sorte à permettre à la puissance mécanique déterminée après l'ajustement de satisfaire à la condition préétablie, les un ou plusieurs processeurs (50) réalisent en particulier l'étape suivante :
ajuster la configuration de paramètre de ventilation selon la puissance mécanique, de sorte à permettre à la puissance mécanique déterminée après l'ajustement d'être inférieure ou égale à un seuil de puissance mécanique préétabli, et/ou d'être inférieure ou égale à la puissance mécanique qui est déterminée avant l'ajustement.

**3.** Dispositif d'assistance respiratoire selon la revendication 1, **caractérisé en ce que**, lorsque les un ou plusieurs processeurs (50) réalisent l'étape consistant à ajuster la configuration de paramètre de ventilation selon la puissance mécanique, de sorte à permettre à la puissance mécanique déterminée après l'ajustement de satisfaire à la condition préétablie, les un ou plusieurs processeurs réalisent en particulier l'étape suivante :
ajuster la configuration de paramètre de ventilation selon la puissance mécanique, de sorte à permettre à la puissance mécanique déterminée après l'ajustement d'augmenter, par rapport à la puissance mécanique déterminée avant l'ajustement, où ladite augmentation est inférieure ou égale à un seuil d'augmentation.

4. Dispositif d'assistance respiratoire selon la revendication 1, **caractérisé en ce que**, lorsque les un ou plusieurs processeurs (50) réalisent l'étape consistant à ajuster la configuration de paramètre de ventilation selon la puissance mécanique, de sorte à permettre à la puissance mécanique déterminée après l'ajustement de satisfaire à la condition préétablie, les un ou plusieurs processeurs (50) réalisent en particulier l'étape suivante :
ajuster la configuration de paramètre de ventilation selon la puissance mécanique, de sorte à réduire le plus possible la puissance mécanique déterminée après l'ajustement.

5. Dispositif d'assistance respiratoire selon l'une quelconque des revendications 1 à 4, **caractérisé en ce que**, lorsque les un ou plusieurs processeurs (50) réalisent l'étape consistant à ajuster la configuration de paramètre de ventilation selon la puissance mécanique, de sorte à permettre à la puissance mécanique déterminée après l'ajustement de satisfaire à la condition préétablie, les un ou plusieurs processeurs (50) réalisent en particulier les étapes suivantes :

déterminer plusieurs configurations de paramètre de ventilation optionnelles dans une plage préétablie de la valeur de consigne du au moins un paramètre de ventilation, où le au moins un paramètre de ventilation correspond à la configuration de paramètre de ventilation ;
déterminer la puissance mécanique qui correspond à chaque configuration de paramètre de ventilation optionnelle ; et
déterminer une configuration de paramètre de ventilation optionnelle, qui correspond à la puissance mécanique qui satisfait à la condition préétablie, comme configuration de paramètre de ventilation cible du dispositif d'assistance respiratoire, de sorte à permettre au dispositif d'assistance respiratoire de ventiler le patient selon la configuration de paramètre de ventilation cible.

6. Dispositif d'assistance respiratoire selon l'une quelconque des revendications 1 à 4, **caractérisé en ce que**, les un ou plusieurs processeurs (50) réalisent en outre l'étape suivante :

obtenir un paramètre de surveillance désigné du patient et/ou un paramètre de ventilation désigné du dispositif d'assistance respiratoire ;
lorsque les un ou plusieurs processeurs (50) réalisent l'étape consistant à ajuster la configuration de paramètre de ventilation selon la puissance mécanique, de sorte à permettre à la puissance mécanique déterminée après l'ajustement de satisfaire à une condition préétablie, les un ou plusieurs processeurs (50) réalisent en particulier l'étape suivante :
ajuster la configuration de paramètre de ventilation selon la puissance mécanique, le paramètre de surveillance désigné et le paramètre de ventilation désigné, de sorte à permettre à la puissance mécanique déterminée après l'ajustement de satisfaire à la condition préétablie, lorsque le paramètre de surveillance désigné et le paramètre de ventilation désigné sont respectivement dans une plage sûre préétablie correspondante.

7. Dispositif d'assistance respiratoire selon la revendication 6, **caractérisé en ce que**, lorsque les un ou plusieurs processeurs (50) réalisent l'étape consistant à ajuster la configuration de paramètre de ventilation selon la puissance mécanique, le paramètre de surveillance désigné et le paramètre de ventilation désigné, les un ou plusieurs processeurs (50) réalisent en particulier les étapes suivantes :

ajuster, sur la base d'une configuration de paramètre de ventilation initiale préétablie, la configuration de paramètre de ventilation, selon la puissance mécanique, le paramètre de surveillance désigné et le paramètre de ventilation désigné ; et/ou
ajuster, dans une plage d'ajustement préétablie de la configuration de paramètre de ventilation, la configuration de paramètre de ventilation, selon la puissance mécanique, le paramètre de surveillance désigné et le paramètre de ventilation désigné.

8. Dispositif d'assistance respiratoire selon la revendication 6, **caractérisé en ce que**, lorsque les un ou plusieurs processeurs (50) réalisent l'étape consistant à obtenir le paramètre de surveillance désigné du patient, les un ou plusieurs processeurs (50) réalisent en particulier l'étape suivante :
obtenir le paramètre de surveillance désigné du patient, par le biais d'au moins l'un : du dispositif d'assistance respiratoire et d'un affichage.

9. Dispositif d'assistance respiratoire selon l'une quelconque des revendications 1 à 4, **caractérisé en ce que**, les un ou plusieurs processeurs (50) réalisent en outre les étapes suivantes :

déterminer un état respiratoire du patient, lorsque le dispositif d'assistance respiratoire commence à ventiler le patient ; et

déterminer une configuration de paramètre de ventilation initiale selon l'état respiratoire, de sorte à permettre au dispositif d'assistance respiratoire de ventiler le patient selon la configuration de paramètre de ventilation initiale.

10. Dispositif d'assistance respiratoire selon la revendication 9, **caractérisé en ce que**, lorsque les un ou plusieurs processeurs (50) réalisent l'étape consistant à déterminer une configuration de paramètre de ventilation initiale selon l'état respiratoire, les un ou plusieurs processeurs (50) réalisent en outre les étapes suivantes :

déterminer une compliance et/ou une résistance des voies respiratoires du système respiratoire du patient ; et déterminer la configuration de paramètre de ventilation initiale selon au moins l'une de la compliance et de la résistance des voies respiratoires.

11. Dispositif d'assistance respiratoire selon l'une quelconque des revendications 1 à 4, **caractérisé en ce que**, les un ou plusieurs processeurs (50) réalisent en outre l'étape suivante :

normaliser la puissance mécanique, qui est déterminée selon la configuration de paramètre de ventilation ; où lorsque les un ou plusieurs processeurs (50) réalisent l'étape consistant à ajuster la configuration de paramètre de ventilation selon la puissance mécanique, les un ou plusieurs processeurs (50) réalisent en outre l'étape suivante :
ajuster la configuration de paramètre de ventilation selon la puissance mécanique normalisée.

12. Dispositif d'assistance respiratoire selon la revendication 11, **caractérisé en ce que**, lorsque les un ou plusieurs processeurs (50) réalisent l'étape consistant à normaliser la puissance mécanique, qui est déterminée selon la configuration de paramètre de ventilation, les un ou plusieurs processeurs (50) réalisent en outre l'étape suivante :
normaliser la puissance mécanique, qui est déterminée selon la configuration de paramètre de ventilation, selon au moins un aspect parmi le poids corporel, la capacité vitale, le volume de ventilation d'une durée préétablie actuelle, la compliance du système respiratoire, et le volume résiduel fonctionnel, du patient.

Obtaining a ventilation parameter configuration of the respiratory support device during mechanical ventilation for a patient, wherein the ventilation parameter configuration comprises a set value for at least one ventilation parameter ~S110

Determining mechanical power according to the ventilation parameter configuration, wherein the mechanical power is configured to indicate power of the mechanical ventilation, which acts on a lung or a respiratory system ~S120

Adjusting the ventilation parameter configuration according to the mechanical power, so as to enable the mechanical power determined after the adjusting to satisfy a preset condition ~S130

FIG. 1

10
Gas flow supply apparatus

20

40
30
Ventilation parameter

50
Processor

60
Memory

Patient

FIG. 2

Expiratory branch 213a

213c

Patient

211

Inspiratory branch 213b

214a

214b

212

G
a
s

Memory 60 — Processor 50 — Display 70

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3238110 A1